Europäisches Patentamt

(19) European Patent Office

Office européen des brevets

(11) Veröffentlichungsnummer: **0 066 770**
**B1**

# (12) EUROPÄISCHE PATENTSCHRIFT

(45) Veröffentlichungstag der Patentschrift:
**11.07.84**

(21) Anmeldenummer: **82104535.8**

(22) Anmeldetag: **25.05.82**

(51) Int. Cl.³: **C 07 C 51/367,** C 07 C 65/03, C 07 C 143/52

(54) **Verfahren zur Herstellung von 3-Hydroxybenzoesäure.**

(30) Priorität: **04.06.81 DE 3122264**
**04.06.81 DE 3122260**

(43) Veröffentlichungstag der Anmeldung:
**15.12.82 Patentblatt 82/50**

(45) Bekanntmachung des Hinweises auf die Patenterteilung:
**11.07.84 Patentblatt 84/28**

(84) Benannte Vertragsstaaten:
**CH DE FR GB LI**

(56) Entgegenhaltungen:
**EP - A - 0 011 815**

(73) Patentinhaber: **BAYER AG, Konzernverwaltung RP Patentabteilung, D-5090 Leverkusen 1 Bayerwerk (DE)**

(72) Erfinder: **Blank, Heinz Ulrich, Dr., Am Geusfelde 35, D-5068 Odenthal (DE)**
Erfinder: **Goldschmitt, Ernst, Dr., Im Daubental 8, D-4047 Dormagen (DE)**
Erfinder: **Marzolph, Gerhard, Dr., Roggendorfstrasse 65, D-5000 Köln 80 (DE)**
Erfinder: **Dürholz, Friedrich, Dr., Knusthöhe 40, D-5630 Remscheid 11 (DE)**
Erfinder: **Mentzel, Werner, Dr., Morgengraben 5, D-5000 Köln 80 (DE)**
Erfinder: **Busse, Roland, Dipl.-Ing., Fasanenweg 19, D-5090 Leverkusen 3 (DE)**
Erfinder: **Gabel, Eike, Dr., Eichenhainallee 14, D-5060 Berg.-Gladbach 1 (DE)**
Erfinder: **Behre, Horst, Dr., Zur alten Linde 12, D-5068 Odenthal (DE)**
Erfinder: **Mayer, Dietmar, Dr., Hamburger Strasse 51, D-5090 Leverkusen 3 (DE)**

## Beschreibung

Die vorliegende Erfindung betrifft ein Verfahren zur Herstellung von 3-Hydroxybenzoesäure aus einem technischen 3-Sulfobenzoesäure-Sulfiergemisch.

Es ist bekannt, 3-Hydroxybenzoesäure mit Hilfe einer Alkalihydroxidschmelze aus 3-Sulfobenzoesäure herzustellen, wobei die Sulfogruppe gegen eine Hydroxygruppe ausgetauscht wird. Zur Alkalischmelze wird im allgemeinen das Mononatriumsalz der 3-Sulfobenzoesäure eingesetzt, das nach Sulfonierung von Benzoesäure mit 2,6 Mol $SO_3$ in Form von 20%igem Oleum pro Mol Benzoesäure, Verdünnen des Sulfonierungsansatzes mit Wasser, partieller Neutralisation mit Natronlauge oder Aussalzen mit Kochsalz, Filtration und Trocknung gewonnen wird [Lieb. Ann. Chem. 280, 6 (1894)]. In J. Chem. Soc. 1950, 2108 wird die Verbesserung eines seit langem bekannten Schmelzverfahrens mit Kaliumhydroxid beschrieben, wobei das Mononatriumsalz der 3-Sulfobenzoesäure mit 50%iger wässriger Natriumhydroxidlösung verrieben wird, das Gemisch getrocknet wird und in Form dieser Mischschmelze mit Kaliumhydroxid verschmolzen wird.

Aus US 3 094 558 ist bekannt, dass der Gebrauch der teuren Kalilauge durch Natriumhydroxid bei gleich guten Ausbeuten ersetzt werden kann, wenn die Umsetzung in Gegenwart kleiner Mengen Wasser durchgeführt wird.

Bei allen bisher bekannten Verfahren wurde die Zwischenisolierung der 3-Sulfobenzoesäure als Mononatriumsalz für notwendig gehalten, weil dieses Salz als Feststoff auf einfache und übersichtliche Weise mit dem Alkalihydroxid gemischt werden kann und weil bei der Zwischenisolierung gleichzeitig eine Reinigung durchgeführt wird, bei der vor allem Schwefelsäure aus dem überschüssigen Sulfierreagenz und ein Teil der bei jeder Sulfierung nach einem wirtschaftlichen Sulfierverfahren unvermeidlichen Nebenprodukte, beispielsweise 2-Sulfobenzoesäure, 4-Sulfobenzoesäure und andere organische Nebenprodukte, in der Mutterlauge in Lösung bleiben.

Diese Verwendung des Mononatriumsalzes der 3-Sulfobenzoesäure in den beschriebenen Verfahren hat jedoch die folgenden Nachteile:

1. Es ist ein zusätzlicher Verfahrensschritt erforderlich;
2. die Zwischenisolierung und die Trocknung bedingen den Einsatz und die nachträgliche Verdampfung grosser Mengen an Wasser und den zugehörigen Energieaufwand;
3. in der Mutterlauge verbleiben beträchtliche Mengen an 3-Sulfobenzoesäure, die die Gesamtausbeute vermindern;
4. das Zwischenisolierungsverfahren bedingt die Entstehung eines Abwasserablaufs, der im Falle der teilweisen Neutralisation und besonders im Falle der Aussalzung mit Kochsalz beträchtliche Mengen Schwefelsäure enthält (Dünnsäure);
5. die im rohen Sulfonierungsgemisch der Benzoesäure enthaltene Wärmeenergie, insbesondere auch die potentielle chemische Energie, die beispielsweise durch Neutralisation freigesetzt wird, geht vollständig verloren.

Es wurde nun ein Verfahren zur Herstellung von 3-Hydroxybenzoesäure durch Umsetzen von 3-Sulfobenzoesäure mit Alkalihydroxid bei erhöhten Temperaturen und gegebenenfalls erhöhtem Druck gefunden, das dadurch gekennzeichnet ist, dass man

a) ein technisches, Schwefelsäure und/oder Schwefeltrioxid enthaltendes 3-Sulfobenzoesäure-Gemisch, das mindestens 75 Gew.-% 3-Sulfobenzoesäure, bezogen auf insgesamt vorhandene organische Bestandteile, und höchstens 35 Gew.-% Schwefelsäure und/oder $SO_3$, bezogen auf die Gesamtmasse, enthält, in die Reaktion einsetzt,
b) dieses Gemisch, gegebenenfalls als wässrige Lösung, mit soviel 50- bis 100-gew.%igem Alkalihydroxid, wobei der Rest von 50 bis 0 Gew.-% im wesentlichen aus $H_2O$ besteht, bei erhöhter Temperatur und gegebenenfalls erhöhtem Druck vermischt, dass nach Neuralisation der Schwefelsäure und der gesamten Sulfo- und Carboxygruppen pro Mol 3-Sulfobenzoesäure 2,5 bis 8 Mol Alkalihydroxid vorliegen,
c) die alkalische Reaktionsmischung in an sich bekannter Weise bei Temperaturen im Bereich von 220 bis 450°C und bei einem Druck von 1 bis 120 bar umsetzt, gegebenenfalls unter Druck soviel Wasser zusetzt, dass im Ansatz 10 bis 45 Gew.-% Wasser vorliegen, und
d) die alkalische Reaktionsmischung, gegebenenfalls nach Verdünnen mit Wasser, mit Mineralsäuren auf einen pH-Wert von kleiner als 4 ansäuert und die 3-Hydroxybenzoesäure danach in an sich bekannter Weise bei Temperaturen im Bereich von –5° bis +40°C isoliert.

Die erfindungsgemäss einsetzbaren technischen Sulfiergemische haben beispielsweise die folgende Zusammensetzung:

70 bis 95 Gew.-% 3-Sulfobenzoesäure,
2,5 bis 7 Gew.-% 4-Sulfobenzoesäure,
0,5 bis 1,5 Gew.-% 2-Sulfobenzoesäure,
0,01 bis 0,5 Gew.-% 3,5-Disulfobenzoesäure,
0,01 bis 1,5 Gew.-% Diphenylsulfon-Derivate,
0,01 bis 1,5 Gew.-% Benzophenon-Derivate und
2,0 bis 20 Gew.-% $SO_3$ (in Form von $SO_3$ und/oder $H_2SO_4$).

Dieses technische Sulfiergemisch kann gegebenenfalls bis zu gleichen Teilen mit Wasser verdünnt werden.

Viele der bekannten Sulfonierungsverfahren liefern 3-Sulfobenzoesäure-Gemische, die einen für das vorliegende Verfahren unwirtschaftlich hohen Anteil an Sulfonierungsreagenzien enthalten oder die mindestens durch einen ungünstig hohen Anteil an unerwünschten organischen Nebenprodukten ausgezeichnet sind.

So ist es bekannt, Benzoesäure mit der 6-fachen Gewichtsmenge konzentrierter Schwefelsäure in Gegenwart von 10 Gew.-%, bezogen auf die Benzoesäure, an Quecksilbersulfat in einer 45stündigen Reaktion bei 135°C umzusetzen [Ber. dt. Chem. Ges. 40, 2411 (1907)]. Der Einsatz so grosser Mengen an Sulfonierreagenz ist unbefriedigend, da zum einen ein erhöhter Chemikalieneinsatz erforderlich ist und zum andern zur Abtrennung der 3-Sulfobenzoesäure von der grossen Menge überschüssigen Sulfonierreagenz das Reaktionsgemisch verdünnt wird und zum Aussalzen der 3-Sulfobenzoesäure mit einer grösseren Menge an Natriumchlorid versetzt wird, wobei man eine salzhaltige verdünnte Schwefelsäure (Dünnsäure) erhält, die technisch nur äusserst schwierig aufgearbeitet werden kann.

Dieses und das bereits weiter oben genannte Sulfonierungsverfahren ergeben neben der gewünschten 3-Sulfobenzoesäure auch die unerwünschten Isomeren, nämlich die 4-Sulfobenzoesäure und die 2-Sulfobenzoesäure. Bei einer Nacharbeitung dieser Verfahren konnten weiterhin auch 3,5-Disulfobenzoesäure, ferner Diphenylsulfon- und Benzophenonderivate nachgewiesen werden. Ein erheblicher Teil der eingesetzten Benzoesäure wird somit für unerwünschte Nebenreaktionen verbraucht. Eine Abtrennung dieser Nebenprodukte ist äusserst aufwendig und mit Ausbeuteverlusten an 3-Sulfobenzoesäure verbunden.

In J. pr. Chemie (2) 143, 127 (1935) ist die Umsetzung von Benzoesäure mit gasförmigem Schwefeltrioxid beschrieben, wobei nach der dortigen Angabe in 100%iger Selektivität die 3-Sulfobenzoesäure erhalten wurde. Hierbei wurden jedoch nur 80% des zu einem vollständigen Umsatz der Benzoesäure erforderlichen Schwefeltrioxids zugesetzt. Dies zeigt, dass unerwünschte Beiprodukte bevorzugt bei höheren Umsätzen als 80% auftreten. Für ein industrielles Verfahren kommt diese Variante nicht in Frage, da die Abtrennung der überschüssigen Benzoesäure nur umständlich und verlustreich durchgeführt werden kann.

Ein weiteres Verfahren zur Herstellung von 3-Sulfobenzoesäure wird in Ind. Eng. Chem. 45, 2065 (1953) beschrieben, wobei die Benzoesäure mit flüssigem Schwefetrioxid (pro Mol eingesetzte Benzoesäure 1,1 Mol $SO_3$) bei einer Temperatur von 125 bis 140°C vermischt wird und die Nachreaktion bei 130 bis 150°C erfolgt. Die Reaktion bei diesem Verfahren ist nach eigenen Beobachtungen nach 2 bis 4 Stunden vollständig. Bei diesem Sulfierverfahren ist es nachteilig, dass ein grosser Teil der eingesetzten Benzoesäure für die Bildung unerwünschter Nebenprodukte verbraucht wird.

Es wurde nun weiterhin gefunden, dass 3-Sulfobenzoesäure-Gemische mit hohen Gehalten an 3-Sulfobenzoesäure und gleichzeitig niedrigem Gehalt an Schwefelsäure beziehungsweise $SO_3$, die dadurch besonders geeignet für den Einsatz im erfindungsgemässen Verfahren sind, hergestellt werden können, wenn man Benzoesäure mit 0,01–0,5 Mol Schwefelsäure pro Mol Benzoesäure vermischt und dann mit $SO_3$ oder Oleum sulfoniert.

Bei diesen Sulfonierungsgemischen ist eine Abtrennung der 3-Sulfobenzoesäure von den überschüssigen Sulfonierungsreagenzien nicht nötig. Gleichzeitig lässt sich der Gehalt an Benzoephenonderivaten in der Sulfierungsschmelze mindestens um die Hälfte der Werte aus dem Verfahren nach Ind. Eng. Chem. (loc. cit.) senken und so die Verunreinigung im Produkt der Alkalischmelze minimieren.

Eine besondere Variante des erfindungsgemässen Verfahrens ist demnach dadurch gekennzeichnet, dass man Benzoesäure mit 0,01 bis 0,5 Mol Schwefelsäure pro Mol Benzoesäure vermischt und mit 1 bis 1,2 Mol $SO_3$ pro Mol Benzoesäure in Form von gasförmigem $SO_3$ und/oder flüssigem $SO_3$, das 0 bis 35 Gew.-% Schwefelsäure, bezogen auf das Gemisch $H_2SO_4$–$SO_3$, enthalten kann, bei erhöhter Temperatur sulfoniert und das entstehende Reaktionsgemisch den oben genannten Massnahmen b), c) und d) unterwirft.

Die zu sulfonierende Benzoesäure wird hierbei mit 0,01 bis 0,5 Mol, bevorzugt 0,05 bis 0,4 Mol, besonders bevorzugt 0,1 bis 0,3 Mol Schwefelsäure pro Mol Benzoesäure vermischt. Als Sulfonierungsreagenz kann gasförmiges und/oder flüssiges Schwefeltrioxid dienen. Flüssiges Schwefeltrioxid kann hierbei auch in Form von Oleum eingesetzt werden. Als Schwefelsäuregehalt in flüssigem $SO_3$ sei hierbei ein Bereich von beispielsweise 0 bis 35 Gew.-% Schwefelsäure, bezogen auf das Gemisch $H_2SO_4$–$SO_3$, genannt.

Die Sulfonierung wird bei einer Temperatur von beispielsweise 40 bis 170°C durchgeführt. Hierbei wird die Vermischung des Benzoesäure/ Schwefelsäure-Gemisches mit dem Sulfonierungsreagenz bei einer Temperatur von 40 bis 140°C, bevorzugt 40 bis 125°C, durchgeführt und die Reaktion danach im oberen Teil des genannten Tempraturbereiches bis 170°C zu Ende geführt. Die Sulfonierung kann beispielsweise in folgenden Varianten durchgeführt werden:

1. Das Benzoesäure/Schwefelsäure-Gemisch wird bei 40 bis 125°C als inhomogenes Gemisch oder als Schmelze vorgelegt und mit 1 bis 1,2 Mol gasförmigem und/oder flüssigem $SO_3$ pro Mol Benzoesäure, bei flüssigem $SO_3$ vorzugsweise in Form von 100%igem $SO_3$, versetzt. Falls erforderlich, wird hierbei die Temperatur durch äussere Kühlung während der Vermischung auf einen Wert unterhalb von 125°C begrenzt. Die Temperatur sollte 40°C nicht unterschreiten, um noch eine ausreichende Durchmischung des Reaktionsgemisches zu ermöglichen. Auch oberhalb von 125°C werden noch gute Ergebnisse erzielt, jedoch sind solche Temperaturen während der Vermischung nicht bevorzugt. Die Sulfonierung wird sodann bei einer Temperatur von 125 bis 170°C, bevorzugt 130 bis 150°C, zu Ende geführt.

2. Flüssiges Schwefeltrioxid oder Oleum werden in der oben angegebenen Menge vorgelegt und das feste oder flüssige Benzoesäure/Schwefelsäure-Gemisch wird hierin eingetragen. Zu Beginn der Vermischung wird die Temperatur im allgemeinen durch den Siedepunkt des Schwefeltrioxids begrenzt, welches beispielsweise durch totalen Rückfluss im Reaktionsansatz gehalten wird. Bei fortschreitender Reaktion kann auch bereits während der Vermischung eine höhere Temperatur im Bereich von 40 bis 125°C erreicht werden. Ein besonders bevorzugter Bereich für die Vermischung liegt bei 40 bis 90°C. Auch bei dieser Variante wird die Sulfonierung bei 125 bis 170°C, bevorzugt 130 bis 150°C, zu Ende geführt.

Beispielhafte Sulfiergemische, die nach diesen Varianten hergestellt werden können, enthalten 64 bis 93 Gew.-% 3-Sulfobenzoesäure und höchstens 35 Gew.-%, bevorzugt weniger als 25 Gew.-%, besonders bevorzugt weniger als 15 Gew.-% Schwefelsäure/Schwefeltrioxid. Die Umsatzrate beträgt hierbei im allgemeinen mehr als 98%, vielfach über 99%. Die Ausbeute, bezogen auf die umgesetzte Benzoesäure, beträgt für die im Sulfiergemisch enthaltenen Komponenten beispielsweise wie folgt:

|  | % der theoretischen Ausbeute |
|---|---|
| 3-Sulfobenzoesäure | 88–95, bevorzugt 92–95 |
| 4-Sulfobenzoesäure | 4–7, bevorzugt 4–5,5 |
| 2-Sulfobenzoesäure | 0,8–1,5, bevorzugt 0,8–1,3 |
| 3,5-Disulfo-benzoesäure | 0,01–0,5, bevorzugt 0,01–0,3 |
| Benzophenonderivate | 0,01–1,5, bevorzugt 0,01–0,8 |
| Diphenylsulfonderivate | 0,01–1,5, bevorzugt 0,01–1,0 |

Diese Werte bedeuten insbesondere einen höheren Anteil an 3-Sulfobenzoesäure und einen geringeren Anteil an Benzophenonderivaten, verglichen mit bisherigen Sulfonierungsverfahren.

Die Senkung des Anteils an Benzophenonderivaten ist besonders vorteilhaft, wenn die 3-Sulfobenzoesäureschmelze direkt zu einem besonders reinen Folgeprodukt weiterverarbeitet werden und gleichzeitig eine aufwendige Reinigung der Sulfierschmelze vermieden werden soll.

Beispielsweise findet man nach einer Alkalischmelze der nach dem Verfahren aus Ind. Eng. Chem. (loc. cit.) gewonnenen 3-Sulfobenzoesäure in der daraus entstandenen 3-Hydroxybenzoesäure ein unbekanntes und nur schwer abtrennbares Nebenprodukt, das wahrscheinlich neben anderen Produkten aus den Benzophenonderivaten gebildet wird.

Es ist ein wesentliches Kennzeichen des erfindungsgemässen Verfahrens, dass solche Sulfiergemische direkt und ohne Zwischenisolierung der 3-Sulfobenzoesäure eingesetzt werden. Die Sulfiergemische werden mit 50- bis 100-gew.-%igem Alkalihydroxid vermischt, wobei der Rest von 50 bis 0 Gew.-% im wesentlichen aus Wasser besteht. Daneben können die Alkalihydroxide bzw. ihre Lösungen noch beispielsweise die in technischen Alkalihydroxidlösungen vorhandenen geringen Mengen an Carbonaten oder Hydrogencarbonaten enthalten. Bevorzugt wird 60- bis 90%iges, insbesondere 65- bis 80%iges Alkalihydroxid eingesetzt. Als Alkalihydroxid sei beispielsweise Natriumhydroxid und Kaliumhydroxid, bevorzugt Natriumhydroxid, genannt. Die Menge an Alkalihydroxid wird so bemessen, dass nach der Neutralisation der Schwefelsäure und der gesamten Sulfo- und Carboxygruppen pro Mol 3-Sulfobenzoesäure 2,5 bis 8 Mol, bevorzugt 3 bis 6 Mol, besonders bevorzugt 3,5 bis 5,5 Mol, ganz besonders bevorzugt 4 bis 5 Mol Alkalihydroxid vorliegen.

Bei diesem Vorgehen wird die in der Sulfonierungsmasse enthaltene Wärmemenge in den folgenden Verfahrensschritt eingebracht und die Energie, die zum Erwärmen und Aufschmelzen der organischen Komponente andernfalls aufgebracht werden müsste, eingespart. Bei der direkten Vermischung des technischen 3-Sulfobenzoesäure-Sulfiergemisches mit gegebenenfalls wasserhaltigem Alkalihydroxid kommt zusätzliche Wärmeenergie aus der Reaktion, insbesondere der Neutralisation von überschüssiger Schwefelsäure, der Sulfo- und Carboxygruppen in das Reaktionsgemisch, wodurch sich ein äusserst wirtschaftliches Verfahren zur Herstellung der 3-Hydroxybenzoesäure erschliesst.

Die Vermischung von technischer 3-Sulfobenzoesäure-Sulfierschmelze mit gegebenenfalls wasserhaltigem Alkalihydroxid, das über seinen Erstarrungspunkt erwärmt ist, kann auf verschiedene Weise erfolgen.

So kann beispielsweise die gegebenenfalls mit Wasser verdünnte Sulfierschmelze oder das Alkalihydroxid vorgelegt werden und die jeweils andere Komponente zudosiert werden; bevorzugt wird das Alkalihydroxid vorgelegt. Man kann jedoch auch beide Komponenten simultan der Vermischung zuführen. Die Vermischung kann sowohl drucklos als auch unter gleichzeitigem Aufbau eines höheren als atmosphärischen Druckes durchgeführt werden. Beim Aufbau eines höheren Druckes kann dieser beispielsweise durch Abblasen von Wasserdampf auf einen vorher festgelegten Wert begrenzt werden. Hier wird das Gemisch gleichzeitig konzentriert. Bei druckloser Verfahrensführung und bei der Begrenzung des Druckes auf einen vorher festgelegten Wert wird ein Teil der Neutralisationswärme, beispielsweise durch einen Kühler oder durch das Abblasen von Wasserdampf abgeführt. In jedem Falle bleibt aber die Neutralisationswärme zumindest teilweise im System und kann vorteilhaft für die folgende Druckhydrolyse ausgenutzt werden.

Unter den möglichen Varianten der Vermischung durch Kombination der genannten Parameter seien beispielsweise die folgenden näher ausgeführt:

1. Die gegebenenfalls mit Wasser verdünnte, bevorzugt unverdünnte, technische 3-Sulfobenzoesäureschmelze wird beispielsweise in auf Siedetemperatur erhitzte, vorgelegte 60- bis 90%ige Natronlauge dosiert, wobei ohne äusserliche Wärmezufuhr kontinuierlich Wasser abdestilliert wird und der Ansatz dadurch konzentrierter wird. Eine Wasserdestillation kann sich aber auch an die Vorvermischung anschliessen.

2. Die Sulfobenzoesäureschmelze wird unter Druck beispielsweise in vorgelegte, geschmolzene 60- bis 90%ige Natronlauge eingepumpt oder eingedrückt, wobei allein durch Ausnutzung der dabei freiwerdenden Wärmemengen eine für die folgende Umsetzung zur 3-Hydroxybenzoesäure günstige Reaktionstemperatur von beispielsweise 260 bis 370°C erreicht wird. Falls bei dieser Verfahrensvariante eine Begrenzung des gleichzeitig ansteigenden Druckes erzielt werden soll, kann dies durch Abblasen von Wasserdampf geschehen.

3. Die in bevorzugter Weise unverdünnte, technische 3-Sulfobenzoesäureschmelze und die konzentrierte, wässrige Natronlauge werden simultan in ein Mischrohr eingepumpt, wobei das Mischrohr so ausgelegt ist, dass allein durch die bewirkte turbulente Strömung eine vollständige Vermischung erfolgt. Gegebenenfalls kann die Vermischung auch durch Einbauten im Mischrohr unterstützt werden, jedoch ist diese Massnahme nicht unbedingt erforderlich. Beispielsweise können in einem Mischrohr mit 500 mm Länge und 5 mm Durchmesser in einer Stunde 2000 bis 5000 ml technische Sulfierschmelze mit entsprechenden Mengen an Natronlauge vermischt und in das Reaktionsgefäss zur anschliessenden Druckhydrolyse gefördert werden. Auch bei dieser Variante kann drucklos oder bei höherem Druck, gegebenenfalls unter Begrenzung dieses höheren Druckes, gearbeitet werden. Bei geeigneter Wahl der Ausgangstemperaturen der Komponenten, beispielsweise 150 bis 180°C für die Sulfierschmelze und 100 bis 180°C für die Natronlauge, wird am Ende des Mischrohres eine für die Umsetzung zur 3-Hydroxybenzoesäure günstige Reaktionstemperatur von beispielsweise 270 bis 320°C erreicht. Es kann auch vorteilhaft sein, die Vermischung bei einer niedrigeren Temperatur vorzunehmen oder den resultierenden Wasserdampfdruck durch Abblasen von Wasserdampf zu begrenzen. Dieses Verfahren stellt ein teilkontinuierliches Verfahren dar, bei dem die Vermischung im Mischrohr der kontinuierliche Verfahrensteil und die folgende Vervollständigung der Umsetzung in einem Autoklaven den diskontinuierlichen Teil darstellt. Da die Reaktion zwischen der 3-Sulfobenzoesäure und dem Alkalihydroxid im oberen Teil des genannten Temperaturbereichs von 220 bis 450°C jedoch immer schneller wird, kann auch bereits im Mischrohr bei genügend hoher Temperatur, beispielsweise in der Nähe von 400°C und bei entsprechender Druckhaltung, die Umwandlung zur 3-Hydroxybenzoesäure und damit die Gesamtreaktion in kontinuierlicher Form vervollständigt werden.

Sofern nicht die im vorangegangenen Absatz genannte kontinuierliche Arbeitsweise im Mischrohr bei einer hohen Temperatur in der Nähe von etwa 400°C gewählt wird, schliesst sich nach allen übrigen Varianten des erfindungsgemässen Verfahrens an die Vermischung von 3-Sulfobenzoesäureschmelze mit Alkalihydroxid vorteilhaft eine Druckhydrolyse der dann vorliegenden Suspension zum 3-Hydroxybenzoesäure-Dinatriumsalz an.

Die Temperatur für diese Druckhydrolyse liegt beispielsweise bei 220 bis 450°C, bevorzugt 260 bis 370°C. Der Druck kann hierbei 1 bis 120 bar betragen. Vorteilhaft wird eine Druckhydrolyse unter dem systemeigenen Wasserdampfdruck oder Wasserdampfpartialdruck von etwa 5 bis etwa 80 bar, bevorzugt 10 bis 40 bar, durchgeführt.

Höhere Konzentrationen des eingesetzten Alkalihydroxids und/oder höhere Reaktionstemperaturen beschleunigen die Umsetzung zur 3-Hydroxybenzoesäure. Beispielsweise sei für eine Temperatur von 370°C und eine Anfangskonzentration der Natronlauge von 80% eine Reaktionsdauer von etwa 15 Minuten oder darunter genannt, weiterhin bei 290°C und anfangs 80%iger Natronlauge eine Reaktionszeit von etwa 4 Stunden, weiterhin bei 340°C und anfangs 50%iger Natronlauge eine Reaktionszeit von etwa 6 Stunden. Wie bereits oben geschildert, kann jedoch die Reaktionszeit bei noch höherer Temperatur und sehr hohen Alkalihydroxidkonzentrationen auf wenige Minuten beschränkt werden, so dass die Verweilzeit in einem Mischrohr für die Durchführung der Druckhydrolyse ausreichend sein kann.

Aus der DE-OS 28 52 163 ist bekannt, dass bei alkalischen Druckhydrolysen die 4-Hydroxy- und 2-Hydroxybenzoesäure zu Phenol decarboxylieren. Unter den Reaktionsbedingungen des erfindungsgemässen Verfahrens läuft diese Reaktion ebenfalls ab. Es wurde gefunden, dass die Decarboxylierung besonders leicht erfolgt, wenn der gesamte Wassergehalt des Ansatzes etwa 10 bis 45 Gew.-%, bevorzugt 20 bis 41 Gew.-%, beträgt. Daher kann es vorteilhaft sein, diesen Konzentrationsbereich, falls er sich nicht aus der Vermischung der Sulfierschmelze mit dem gegebenenfalls wässrigen Alkalihydroxid von selbst einstellt, durch nachträgliche Wasserzugabe einzustellen, um die Decarboxylierung der genannten Hydroxybenzoesäure zu vervollständigen. Das entstehende Phenol kann im Gegensatz zur 4-Hydroxy- und 2-Hydroxybenzoesäure und auch im Gegensatz zur ursprünglich vorhandenen 4- und 2-Sulfobenzoesäure in einfacher Weise bei der Isolierung der 3-Hydroxybenzoesäure entfernt werden, da es infolge seiner Löslichkeit in der Mutterlauge verbleibt.

Die Aufarbeitung und Isolierung der freien 3-Hydroxybenzoesäure erfolgt nach gebräuchlicher Methode durch Ansäuern mit einer starken Mineralsäure, wie beispielsweise Salzsäure oder Schwefelsäure, wonach das Gemisch mit einer zum Auflösen der anorgansichen Salze notwendigen Menge Wasser verdünnt wird und die gewünschte 3-Hydroxybenzoesäure in hoher Reinheit ausfällt. Für diese Ausfällung wird beispielsweise eine Temperatur von etwa –5°C bis etwa +40°C eingestellt.

Hierzu wurde gefunden, dass man besonders reine Produkte, die frei von organischen Verunreinigungen sind, dann erhält, wenn zur Isolierung der 3-Hydroxybenzoesäure ein pH-Wert von etwa 0 bis etwa 4, bevorzugt 1 bis 3, besonders bevorzugt 2 bis 2,8, eingestellt wird.

Das erfindungsgemässe Verfahren zeichnet sich durch besonders hohe Ausbeuten an 3-Hydroxybenzoesäure aus. Bei der Verwendung eines technischen 3-Sulfobenzoesäure-Sulfiergemisches ist sie überraschenderweise mit 100 bis 101% Reaktionsausbeute, bezogen auf im eingesetzten Sulfiergemisch vorhandene 3-Sulfobenzoesäure, noch höher als beim Einsatz von reiner 3-Sulfobenzoesäure, wobei Ausbeuten an 3-Hydroxybenzoesäure von etwa 96 bis 98% erreicht werden. Obwohl diese Tatsache nicht vollständig geklärt ist, kann angenommen werden, dass Diphenylsulfon- und Benzophenon-Derivate, die als Nebenbestandteile bis zu 3 Gew.-% im 3-Sulfobenzoesäure-Sulfiergemisch vorliegen können, neben der 3-Sulfobenzoesäure ebenfalls zu 3-Hydroxybenzoesäure umgesetzt werden. Damit wird ein Teil der ursprünglich zur Sulfonierung eingesetzten Benzoesäure noch nachträglich zum gewünschten Produkt umgesetzt, während bei Verfahren des Standes der Technik, die eine Zwischenreinigung der 3-Sulfobenzoesäure vornehmen, diese günstige Umwandlung von Nebenprodukten ausgeschlossen ist.

3-Hydroxybenzoesäure ist ein wichtiges Zwischenprodukt zur Herstellung von Pflanzenschutzmitteln. So kann nach US 4 031 131 3-Hydroxybenzoesäure in methanolischer Lösung und in Gegenwart von Kaliumhydroxid und Dimethylsulfoxid mit 3,4-Dichlorbenzotrifluorid zur 3-(2-Chlor-4-trifluormethylphenoxy)-benzoesäure umgesetzt werden. Durch Nitrieren dieser Benzoesäure mit Kaliumnitrat in konzentrierter Schwefelsäure erhält man sodann die 5-(2-Chlor-4-trifluormethylphenoxy)-2-nitrobenzoesäure, die nach US 3 798 276 ein wichtiges Herbizid darstellt.

Beispiel 1
(Herstellung eines 3-Sulfobenzoesäure-Gemisches)

In einem Rührgefäss werden 305 g (2,5 Mol) Benzoesäure und 49 g (0,5 Mol) Schwefelsäure bei 110°C aufgeschmolzen und durch Eingasen mit 210 g (2,63 Mol) $SO_3$ versetzt, wobei die Temperatur auf 120°C durch äusserliche Kühlung begrenzt wird.

Zur Nachreaktion wird 4 Stunden bei 140°C nachgerührt. Man erhält 559 g (99,2% der Gesamtmenge der Einsatzstoffe) 3-Sulfobenzoesäure-Sulfiergemisch, das bei 140°C als viskose Schmelze vorliegt.

Die Schmelze enthält:

468 g  3-Sulfobenzoesäure =
92,6% der theoretischen Ausbeute
24,8 g 4-Sulfobenzoesäure =
4,9% der theoretischen Ausbeute
4,5 g 2-Sulfobenzoesäure =
0,9% der theoretischen Ausbeute
1,9 g Benzophenonderivate =
0,5% der theoretischen Ausbeute
1,1 g Diphenylsulfonderivate =
0,3% der theotetischen Ausbeute
2,4 g nicht umgesetzte Benzoesäure =
0,8% der eingesetzten Benzoesäure

Beispiel 2
In einem Rührgefäss werden 305 g (2,5 Mol) Benzoesäure und 25 g (0,25 Mol) Schwefelsäure bei Raumtemperatur vermischt. 220 g (2,75 Mol) flüssiges $SO_3$ werden zugetropft, wobei durch äussere Kühlung die Temperatur im Gefässinnern auf 40–70°C begrenzt wird.

Nach beendeter Zugabe wird innerhalb 1 Stunde auf 140°C geheizt und 3 Stunden nachgerührt.

Die resultierende Schmelze enthält:

85   Gew.-% 3-Sulfobenzoesäure =
92,5% der theoretischen Ausbeute
4,3 Gew.-% 4-Sulfobenzoesäure =
4,7% der theoretischen Ausbeute
1,0 Gew.-% 2-Sulfobenzoesäure =
1,1% der theoretischen Ausbeute
0,4 Gew.-% Benzophenonderivate =
0,6% der theoretischen Ausbeute
0,2 Gew.-% Diphenylsulfonderivate =
0,3% der theoretischen Ausbeute
0,1 Gew.-% Benzoesäure =
0,2% der eingesetzten Benzoesäure

Beispiel 3
Bei 40°C werden in einem Rührgefäss 310 g 65%iges Oleum (2,52 Mol freies $SO_3$) vorgelegt und mit einem flüssigen Gemisch aus 305 g (2,5 Mol) Benzoesäure und 25 g (0,25 Mol) Schwefelsäure so versetzt, dass die Innentemperatur nicht über 90°C steigt. Die resultierende, gut rührbare Suspension wird innerhalb 1 Stunde auf 130°C erhitzt und dort 4 Stunden nachgerührt. Die Schmelze enthält:

74,2 Gew.-% 3-Sulfobenzoesäure =
94% der theoretischen Ausbeute
3,2 Gew.-% 4-Sulfobenzoesäure =
4,0% der theoretischen Ausbeute
0,6 Gew.-% 2-Sulfobenzoesäure =
0,8% der theoretischen Ausbeute
0,4 Gew.-% Benzophenonderivate =
0,6% der theoretischen Ausbeute
0,4 Gew.-% Diphenylsulfonderivate =
0,6% der theoretischen Ausbeute

Beispiel 4

[Herstellung eines 3-Sulfobenzoesäure-Gemisches nach Ind. Eng. Chem. 45, 2065 (1953)]

In einem Rührgefäss werden 1465 g (12 Mol) Benzoesäure bei 125°C aufgeschmolzen und durch Zutropfen 1056 g (13,2 Mol) flüssiges $SO_3$ zugegeben, wobei die Innentemperatur auf 130°C begrenzt wird. Nach Zugabe wird die Innentemperatur auf 140°C gesteigert und 4 Stunden gehalten. Man erhält 2505 g (99,4 Gew.-% der Gesamtmenge an Einsatzstoffen) Schmelze folgender Zusammensetzung:

88,0 Gew.-% 3-Sulfobenzoesäure =
   90,9% der theoretischen Ausbeute
5,8 Gew.-% 4-Sulfobenzoesäure =
   6,0% der theoretischen Ausbeute
1,0 Gew.-% 2-Sulfobenzoesäure =
   1,0% der theoretischen Ausbeute
0,1 Gew.-% Benzoesäure =
   0,2% der theoretischen Ausbeute
0,8 Gew.-% Benzophenonderivate =
   1,1% der theoretischen Ausbeute
0,5 Gew.-% Diphenylsulfonderivate =
   0,7% der theoretischen Ausbeute

Beispiel 5

224 g 3-Sulfobenzoesäure-Schmelze, die nach Beispiel 1 hergestellt wurde, wird in einem Tropftrichter aufgeschmolzen.

In einem Glaskolben mit Destillationseinrichtung werden 400 g 70%ige Natronlauge (= 7,0 Mol NaOH), die auf 156°C erhitzt und intensiv gerührt wird, vorgelegt.

Die Sulfobenzoesäureschmelze wird innerhalb von 15 Minuten zugetropft, wobei ohne weitere Wärmezufuhr 63 ml $H_2O$ abdestilliert werden.

Die heisse, gut rührbare Suspension wird in einen 0,7 l Ni-Rührautoklaven überführt und 4 Stunden auf 290°C erhitzt, wobei sich ein Druck von 22 bar einstellt. Der Ansatz wird mit 520 ml $H_2O$ verdünnt und mit 37%iger Salzsäure auf pH 2 angesäuert. Das ausgefallene und bei 20°C abfiltrierte Produkt wird mit Wasser salzfrei gewaschen und im Vakuum getrocknet. Man erhält 126,7 g 99%ige 3-Hydroxybenzoesäure = 98% der theoretischen Ausbeute, bezogen auf 3-Sulfobenzoesäure, oder 91% der theoretischen Ausbeute, bezogen auf eingesetzte Benzoesäure.

Weitere Beispiele 5a–5f mit variierten Reaktionsbedingungen sind in der folgenden Tabelle aufgeführt.

| Beispiel | Sulfierschmelze | | | Natronlauge | | $H_2O$ dest. | Druckhydrolyse | | | Ausbeute % der theoretischen Ausbeute, bezogen auf | |
|---|---|---|---|---|---|---|---|---|---|---|---|
| | [g] | Mol BS* | Gew.-% $H_2SO_4+SO_3$ | Mol | $C_{Anfang}$ [Gew.-%] | [ml] | [°C] | Druck [bar] | Zeit [h] | 3-SBS* | BS* |
| 5a | 224 | 1 | 10,6** | 7,0 | 80 | Vermisch. unter Rückfluss | 290 | 22 | 4 | 98 | 91 |
| 5b | 220 | 1 | 8,2*** | 6,4 | 70 | 49 | 290 | 22 | 4 | 98 | 91 |
| 5c | 256 | 1 | 21,3**** | 8,0 | 80 | Vermisch. unter Rückfluss | 300 | 28 | 4 | 98 | 92 |
| 5d | 256 | 1 | 21,3***** | 8,0 | 70 | 93 | 290 | 22 | 4 | 98 | 89 |
| 5e | 210 | 1 | 3,5+ | 6,6 | 50 | 91 | 300 | 32 | 4 | 98 | 90 |
| 5f | 210 | 1 | 3,5+ | 5,1 | 80 | Vermisch. unter Rückfluss | 290 | 22 | 4 | 97 | 88 |

\*     BS = Benzoesäure; 3-SBS = 3-Sulfobenzoesäure
\*\*    3-Sulfobenzoesäure-Sulfiergemisch aus Beispiel 1
\*\*\*   3-Sulfobenzoesäure-Sulfiergemisch aus Beispiel 2
\*\*\*\* 3-Sulfobenzoesäure-Sulfiergemisch aus Beispiel 3
\*\*\*\*\* 3-Sulfobenzoesäure-Sulfiergemisch wie nach Beispiel 3 erhalten, jedoch durch Vorlegen der BS und Zugabe von Oleum 65%
\+      3-Sulfobenzoesäure-Sulfiergemisch aus Beispiel 4

Beispiel 6

In einem 3-l-Nickelrührautoklaven werden 1900 g 80%ige Natronlauge (= 38 Mol NaOH) bei 240°C und 2 bar Eigendruck vorgelegt.

Mit Hilfe einer Zahnraddosierpumpe werden 1194 g (5,43 Mol eingesetzte Benzoesäure) der 3-Sulfobenzoesäure aus Beispiel 2 als 180°C heisse Schmelze innerhalb von 20 Minuten unter die Oberfläche der intensiv gerührten Natronlauge gepumpt. Nach vollständiger Zugabe ist die Temperatur auf 289°C, der Druck auf 21 bar gestiegen. Bei 290°C wird 4 Stunden nachgerührt, der Ansatz mit insgesamt 3000 ml $H_2O$ verdünnt und mit 37%iger HCl auf pH 1 angesäuert.

Das ausgefallene und bei 20°C abfiltrierte Produkt wird mit insgesamt 1200 ml Wasser gewaschen und getrocknet. Man erhält 693 g 98,5%ige 3-Hydroxybenzoesäure = 98% der theoretischen Ausbeute, bezogen auf 3-Sulfobenzoesäure, oder 91% der theoretischen Ausbeute, bezogen auf eingesetzte Benzoesäure.

Beispiel 7

In einem 1,3-l-Nickelautoklaven werden bei 330°C und einem Druck von 11 bar 825 g 80%ige Natronlauge (= 16,5 Mol NaOH) vorgelegt. Innerhalb von 30 Sekunden werden ca. 550 g (2,5 Mol eingesetzte Benzoesäure) der 3-Sulfobenzoesäure aus Beispiel 2 als 180°C heisse Schmelze mit Hilfe von 60 bar Stickstoff über eine beheizte Leitung unter die Oberfläche der intensiv gerührten Natronlauge gedrückt.

Die Temperatur steigt momentan auf 378°C, der Druck auf 59 bar an. Eine nachfolgende Analyse gibt an, dass 84% = 2,1 Mol der Sulfierschmelze hierbei zur Reaktion gebracht werden. Nach 15-minütiger Reaktionszeit bei 370°C und einem bis auf 65 bar steigenden Druck beträgt der Umsatz der 3-Sulfobenzoesäure über 99%. Das Reaktionsgemisch wird durch Zupumpen von $H_2O$ abgekühlt und verdünnt. Die Aufarbeitung erfolgt wie in den vorstehenden Beispielen.

Man erhält 266 g 98%ige 3-Hydroxybenzoesäure = 97% der theoretischen Ausbeute, bezogen auf eingedrückte 3-Sulfobenzoesäure, oder 90% der theoretischen Ausbeute, bezogen auf ursprünglich eingesetzte Benzoesäure.

Beispiel 8

Geschmolzene, 180°C heisse, technische 3-Sulfobenzoesäure, die aus 12 Mol Benzoesäure, 1,2 Mol $H_2SO_4$ und 13,2 Mol $SO_3$ hergestellt wurde (Zusammensetzung wie in Beispiel 2) und 100°C heisse, 75%ige Natronlauge, werden aus 2 Vorratsgefässen mit Hilfe von 2 Zahnraddosierpumpen, deren Förderleistung so eingestellt ist, dass auf die Menge Sulfierschmelze, die 1 Mol Benzoesäure enthält, 7 Mol NaOH als 75%ige Natronlauge gefördert werden, in ein Strömungsrohr simultan eingepumpt. Das Strömungsrohr ist aus Nickel und hat einen Innendurchmesser von 5 mm und eine Länge von 500 mm. Während des Durchströmens des Rohres haben sich Sulfierschmelze und Natronlauge vollständig vermischt und neutralisiert. Das Gemisch gelangt mit einer Temperatur von 270°C in einen Nickelautoklaven, in dem bei 210°C und 5 bar Druck 400 g 50%ige Natronlauge (= 5 Mol NaOH) vorgelegt sind. Durch Abblasen von Wasserdampf wird der Druck während der Zugabe auf 7 bar begrenzt.

Nach Förderung von 6,4 Mol Benzoesäure in Form von 3-Sulfobenzoesäureschmelze und etwa 45 Mol Natronlauge wird die Förderung unterbrochen.

Die Endtemperatur im Nickelautoklaven beträgt 240°C, der Druck 7 bar.

Die Umsetzung der 3-Sulfobenzoesäure ist nach einer Druckhydrolyse bei 300°C und 28 bar in 4 Stunden vollständig. Die Aufarbeitung erfolgt wie in den Beispielen 5 und 6.

Man erhält 803 g 98%ige 3-Hydroxybenzoesäure = 89% der theoretischen Ausbeute, bezogen auf eingesetzte Benzoesäure.

Beispiel 9 (Vergleichsbeispiel)

Sulfonierung: nach Ann. Chem. 280, 6 (1894) mit Zwischen-Isolierung zur Entfernung von $H_2SO_4$; Druckhydrolyse: nach US 3 094 558, Beispiel 3.

500 g Benzoesäure werden innerhalb von 10 Minuten mit 1000 g 20%igem Oleum versetzt und in 120 Minuten auf 210°C erhitzt. Nach 4 Stunden Rühren löst sich eine Probe vollständig in Wasser, die Umsetzung ist somit vollständig. Man erhält eine dunkle, fast schwarze Schmelze. Das Rohgemisch (1456 g = 97% der Gesamtmenge der Einsatzstoffe) enthält neben überschüssiger Schwefelsäure:

51,8 Gew.-% 3-Sulfobenzoesäure ≙ 91,0% der theoretischen Ausbeute

3,6 Gew.-% 4-Sulfobenzoesäure ≙ 6,3% der theoretischen Ausbeute

0,4 Gew.-% 2-Sulfobenzoesäure ≙ 0,9% der theoretischen Ausbeute

0,4 Gew.-% 3,5 Disulfobenzoesäure ≙ 0,5% der theoretischen Ausbeute

0,2 Gew.-% Benzoesäure ≙ 0,6% der theoretischen Ausbeute

0,04 Gew.-% Benzophenonderivate ≙ 0,1% der theoretischen Ausbeute

0,3 Gew.-% Diphenylsulfonderivate ≙ 0,7% der theoretischen Ausbeute

Der Ansatz wird mit 2000 ml $H_2O$ verdünnt, mit 2200 ml gesättigter Kochsalzlösung und zusätzlich 66 g NaCl versetzt. Bei Raumtemperatur wird der Niederschlag abfiltriert, abgepresst und mit gesättigter NaCl-Lösung gewaschen. Man erhält nach Trocknen 981 g 3-Sulfobenzoesäure-Na-Salz mit 75,8 Gew.-% 3-Sulfobenzoesäure (Angabe als freie Säure) (Ausbeute: 3-Sulfobenzoesäure-Na-Salz = 89,7% der theoretischen Ausbeute, bezogen auf Benzoesäure).

296 g des zwischenisolierten Produkts = 1 Mol 3-Sulfobenzoesäure-Na-Salz werden mit 200 g NaOH und 67,5 g $H_2O$ in einem Ni-Autoklaven für 5 Stunden 20 Minuten unter eigenem Wasserdampfdruck auf 300°C erhitzt und wie üblich aufgearbeitet. Man erhält 129,6 g 99%ige 3-Hyroxybenzoesäure = 94% der theoretischen Ausbeute, bezogen auf eingesetzte 3-Sulfobenzoesäure bzw. 84 % der theoretischen Ausbeute, bezogen auf ursprünglich eingesetzte Benzoesäure.

**Patentansprüche**

1. Verfahren zur Herstellung von 3-Hydroxybenzoesäure durch Umsetzen von 3-Sulfobenzoesäure mit Alkalihydroxid bei erhöhten Temperaturen und gegebenenfalls erhöhtem Druck, dadurch gekennzeichnet, dass man

a) ein technisches, Schwefelsäure und/oder Schefeltrioxid enthaltendes 3-Sulfobenzoesäure-Gemisch, das mindestens 75 Gew.-% 3-Sulfobenzoesäure, bezogen auf insgesamt vorhandene organische Bestandteile, und höchstens 35 Gew.-% Schwefelsäure und/oder SO₃, bezogen auf die Gesamtmasse, enthält, in die Reaktion einsetzt,
b) dieses Gemisch, gegebenenfalls als wässrige Lösung, mit soviel 50- bis 100-gew.-%igem Alkalihydroxid, wobei der Rest von 50 bis 0 Gew.-% im wesentlichen aus Wasser besteht, bei erhöhter Temperatur und gegebenenfalls erhöhtem Druck vermischt, dass nach Neutralisation der Schwefelsäure und der gesamten Sulfo- und Carboxygruppen pro Mol 3-Sulfobenzoesäure 2,5 bis 8 Mol Alkalihydroxid vorliegen,
c) die alkalische Reaktionsmischung in an sich bekannter Weise bei Temperaturen im Bereich von 220 bis 450°C und bei einem Druck von 1 bis 120 bar umsetzt, gegebenenfalls unter Druck soviel Wasser zusetzt, dass im Ansatz 10 bis 45 Gew.-% Wasser vorliegen, und
d) die alkalische Reaktionsmischung, gegebenenfalls nach Verdünnen mit Wasser, mit Mineralsäuren auf einen pH-Wert von kleiner als 4 ansäuert und die 3-Hydroxybenzoesäure danach in an sich bekannter Weise bei Temperaturen im Bereich von –5°C bis +40°C isoliert.

2. Verfahren nach Anspruch 1, dadurch gekennzeichnet, dass man ein technisches 3-Sulfobenzoesäure-Gemisch einsetzt, das

70 bis 95 Gew.-% 3-Sulfobenzoesäure,
2,5 bis 7 Gew.-% 4-Sulfobenzoesäure,
0,5 bis 1,5 Gew.-% 2-Sulfobenzoesäure,
0,01 bis 0,5 Gew.-% 3,5-Di-sulfobenzoesäure,
0,01 bis 1,5 Gew.-% Diphenylsulfon-Derivate,
0,01 bis 1,5 Gew.-% Benzophenon-Derivate und
2 bis 20 Gew.-% SO₃ (in Form von SO₃ und/oder H₂SO₄),

bezogen auf die Gesamtmasse, enthält und dieses Gemisch gegebenenfalls bis zu gleichen Teilen mit Wasser verdünnt.

3. Verfahren nach Anspruch 1, dadurch gekennzeichnet, dass man Benzoesäure mit 0,01 bis 0,5 Mol Schwefelsäure pro Mol Benzoesäure vermischt und mit 1 bis 1,2 Mol SO₃ pro Mol Benzoesäure in Form von gasförmigem und/oder flüssigem SO₃, das 0 bis 35 Gew.-% Schwefelsäure, bezogen auf das Gemisch H₂SO₄–SO₃ enthalten kann, bei erhöhter Temperatur sulfoniert und das entstehende Reaktionsgemisch den Massnahmen b), c) und d) unterwirft.

4. Verfahren nach Anspruch 3, dadurch gekennzeichnet, dass man das Benzoesäure/Schwefelsäure-Gemisch vorlegt, bei 40 bis 125°C gasförmiges und/oder flüssiges SO₃, das 0 bis 35 Gew.-% Schwefelsäure, bezogen auf das Gemisch H₂SO₄–SO₃, enthalten kann, zudosiert, die Sulfonierung bei 125 bis 170°C zu Ende führt und das entstehende Reaktionsgemisch den Massnahmen b), c) und d) unterwirft.

5. Verfahren nach Anspruch 3, dadurch gekennzeichnet, dass man flüssiges SO₃, das 0 bis 35 Gew.-% Schwefelsäure, bezogen auf das Gemisch H₂SO₄–SO₃, enthalten kann, vorlegt, das Benzoesäure/Schwefelsäure-Gemisch bei 40 bis 125°C zudosiert, die Sulfonierung bei 125 bis 170°C zu Ende führt und das entstehende Reaktionsgemisch den Massnahmen b), c) und d) unterwirft.

6. Verfahren nach Ansprüchen 1 bis 5, dadurch gekennzeichnet, dass man während oder gegebenenfalls nach der drucklosen Vermischung des 3-Sulfobenzoesäuregemisches mit Alkalihydroxid Wasser abdestilliert oder den Druck bei der Vermischung durch Wasserdestillation begrenzt.

7. Verfahren nach Ansprüchen 1 bis 5, dadurch gekennzeichnet, dass man die Vermischung des 3-Sulfobenzoesäuregemisches mit Alkalihydroxid zur Aufrechterhaltung des entstehenden Druckes im geschlossenen Reaktor durchführt.

8. Verfahren nach Anspruch 7, dadurch gekennzeichnet, dass man als Reaktor ein Mischrohr einsetzt.

9. Verfahren nach Ansprüchen 1 bis 8, dadurch gekennzeichnet, dass man die Druckhydrolyse der alkalischen Reaktionsmischung unter dem systemeigenen Wasserdampfdruck oder Wasserdampfpartialdampfdruck von etwa 5 bis 80 bar durchführt.

10. Verfahren nach Ansprüchen 1 bis 9, dadurch gekennzeichnet, dass man das nach der Druckhydrolyse vorliegende Gemisch mit Mineralsäuren auf einen pH-Wert von etwa 0 bis 4 ansäuert und mit der zur Lösung der anorganischen Salze notwendigen Menge Wasser verdünnt.

**Claims**

1. Process for the preparation of 3-hydroxybenzoic acid by reacting 3-sulphobenzoic acid with an alkali metal hydroxide at elevated temperatures and, if appropriate, elevated pressure, characterised in that

a) a technical 3-sulphobenzoic acid mixture containing sulphuric acid and/or sulphur trioxide, which contains at least 75% by weight of 3-sulphobenzoic acid, relative to the total organic constituents present and not more than 35% by weight of sulphuric acid and/or SO₃, relative to the total mass, is employed in the reaction,
b) this mixture, if appropriate as an aqueous solution, is mixed at elevated temperature and, if appropriate, elevated pressure, with sufficient 50 to 100% strength by weight alkali metal hydroxide, the remaining 50 to 0% by weight consisting essentially of water, for 2.5 to 8 mols of alkali metal hydroxide to be present per mol of 3-sulphobenzoic acid, after neutralisation of the sulphuric acid and all the sulpho and carboxyl groups,
c) the alkaline reaction mixture is reacted in a manner which is in itself known, at temperatures within the range from 220 to 450°C and under a pressure of 1 to 120 bars, if appropriate sufficient water is added under pressure for 10 to 45% by weight of water to be present in the mixture, and
d) the alkaline reaction mixture, if appropriate af-

**0 066 770**

ter dilution with water is acidified with mineral acids to a pH value less than 4 and the 3-hydroxybenzoic acid is then isolated in a manner which is in itself known at temperatures within the range from –5°C to +40°C.

2. Process according to Claim 1, characterised in that a technical 3-sulphobenzoic acid mixture containing, relative to the total mass: 70 to 95% by weight of 3-sulphobenzoic acid, 2.5 to 7% by weight of 4-sulphobenzoic acid, 0.5 to 1.5% by weight of 2-sulphobenzoic acid, 0.01 to 0.5% by weight of 3,5-disulfphobenzoic acid, 0.01 to 1.5% by weight of diphenyl sulphone derivatives, 0.01 to 1.5% by weight of benzophenone derivatives and 2 to 20% by weight of $SO_3$ (in the form of $SO_3$ and/or $H_2SO_4$), is employed and, if appropriate, this mixture is diluted with water up to equal parts.

3. Process according to Claim 1, characterised in that benzoic acid is mixed with 0.01 to 0.5 mol of sulphuric acid per mol of benzoic acid and is sulphonated at elevated temperature with 1 to 1.2 mols of $SO_3$ per mol of benzoic acid, in the form of gaseous and/or liquid $SO_3$ which can contain 0 to 35% by weight of sulphuric acid, relative to the $H_2SO_4$–$SO_3$ mixture, and the resulting reaction mixture is subjected to measures b), c) and d).

4. Process according to Claim 3, characterised in that the benzoic acid/sulphuric acid mixture is initially introduced, gaseous and/or liquid $SO_3$ which can contain 0 to 35% by weight of sulphuric acid, relative to the $H_2SO_4$–$SO_3$ mixture, is metered in at 40 to 125°C, the sulphonation is completed at 125 to 170°C and the resulting reaction mixture is subjected to measures b), c) and d).

5. Process according to Claim 3, characterised in that liquid $SO_3$ which can contain 0 to 35% by weight of sulphuric acid, relative to the $H_2SO_4$–$SO_3$ mixture, is initially introduced, the benzoic acid/sulphuric acid mixture is metered in at 40 to 125°C, the sulphonation is completed at 125 to 170°C and the resulting reaction mixture is subjected to measures b), c) and d).

6. Process according to Claims 1 to 5, characterised in that, during or, if appropriate, after mixing the 3-sulphobenzoic acid mixture with an alkali metal hydroxide at normal pressure, water is removed by distillation or the pressure during the mixing operation is limited by distilling off water.

7. Proces according to Claims 1 to 5, characterised in that the 3-sulphobenzoic acid mixture is mixed with an alkali metal hydroxide in a closed reactor in order to maintain the pressure which is formed.

8. Process according to Claim 7, characterised in that a mixing tube is employed as the reactor.

9. Process according to Claims 1 to 8, characterised in that the pressure hydrolysis of the alkaline reaction mixture is carried out under the autogenous steam pressure of the system or a partial steam pressure of about 5 to 80 bars.

10. Process according to Claims 1 to 9, characterised in that the mixture present after the pressure hydrolysis is acidified with mineral acids to a pH value of about 0 to 4 and is diluted with the quantity of water required to dissolve the inorganic salts.

**Revendications**

1. Procédé de préparation de l'acide 3-hydroxybenzoïque par réaction de l'acide 3-sulfobenzoïque avec un hydroxyde alcalin à des températures élevées et éventuellement sous pression élevée, procédé caractérisé en ce que:

a) on fait réagir un mélange technique comportant de l'acide 3-sulfobenzoïque, et contenant de l'acide sulfurique et/ou du trioxyde de soufre, mélange qui contient au moins 75% en poids d'acide 3-sulfobenzoïque, par rapport aux constituants organiques totaux présents, et au maximum 35% en poids d'acide sulfurique et/ou de $SO_3$, par raport à la masse globale,

b) on incorpore à ce mélange, éventuellement sous forme d'une solution aqueuse, une quantité d'hydroxyde alcalin à 50 à 100% en poids (le reste, de 50 à 0% en poids, consistant essentiellement en de l'eau), à température élevée et éventuellement sous pression élevée, telle qu'après neutralisation de l'acide sulfurique et de l'ensemble des groupes sulfo et carboxyles il reste, par mole d'acide 3-sulfobenzoïque, 2,5 à 8 moles de l'hydroxyde alcalin,

c) on fait réagir le mélange réactionnel alcalin, de façon connue en elle-même, à des températures comprises entre 220°C et 450°C et sous une pression de 1 à 120 bars, et l'on ajoute éventuellement sous pression une quantité d'eau telle qu'il y ait dans la charge 10 à 45% en poids d'eau, et

d) on acidifie le mélange réactionnel alcalin, éventuellement après dilution à l'eau, avec des acides minéraux jusqu'à un pH inférieur à 4, puis l'on isole, de façon connue en soi, l'acide 3-hydroxybenzoïque à des températures comprises entre –5°C et +40°C.

2. Procédé selon la revendication 1, caractérisé en ce qu'on utilise un mélange technique contenant de l'acide 3-sulfobenzoïque et qui contient:

70 à 95% en poids d'acide 3-sulfobenzoïque,
2,5 à 7% en poids d'acide 4-sulfobenzoïque,
0,5 à 1,5% en poids d'acide 2-sulfobenzoïque,
0,01 à 0,5% en poids d'acide 3,5-di-sulfobenzoïque,
0,01 à 1,5% en poids de dérivés de la diphénylsulfone,
0,01 à 1,5% en poids de dérivés de la benzophénone, et
2 à 20% en poids de $SO_3$ (sous forme de $SO_3$ et/ou de $H_2SO_4$),
par rapport à la masse totale, et l'on dilue ce mélange à l'eau en ajoutant éventuellement jusqu'à des parties égales.

3. Procédé selon la revendication 1, caractérisé en ce qu'on mélange l'acide benzoïque avec 0,01 à 0,5 mole d'acide sulfurique par mole d'acide benzoïque, on sulfone à température élevée avec, par mole d'acide benzoïque, 1 à 1,2 mole de

SO$_3$ sous forme de SO$_3$ gazeux et/ou liquide, qui peut contenir 0 à 35% en poids d'acide sulfurique, par rapport au mélange H$_2$SO$_4$–SO$_3$, et l'on soumet le mélange réactionnel résultant aux mesures b), c) et d).

4. Procédé selon la revendication 3, caractérisé en ce qu'on place tout d'abord le mélange acide benzoïque/acide sulfurique, on y ajoute à 40 à 125°C du SO$_3$ gazeux et/ou liquide, qui peut contenir 0 à 35% en poids d'acide sulfurique, par rapport au mélange H$_2$SO$_4$–SO$_3$, on conduit la sulfonation à son terme à 125 à 170°C et l'on soumet le mélange réactionnel résultant aux mesures b), c) et d).

5. Procédé selon la revendication 3, caractérisé en ce qu'on place tout d'abord du SO$_3$ liquide, qui peut contenir 0 à 35% en poids d'acide sulfurique, par rapport au mélange H$_2$SO$_4$–SO$_3$, on introduit de façon dosée le mélange acide benzoïque/acide sulfurique à 40 à 125°C, on conduit la sulfonation à son terme à 125 à 170°C et l'on soumet le mélange réactionnel résultant aux mesures b), c) et d).

6. Procédé selon les revendications 1 à 5, caractérisé en ce que, pendant ou éventuellement après le mélangeage sans pression du mélange contenant de l'acide 3-sulfobenzoïque avec de l'hydroxyde alcalin, on chasse l'eau par distillation ou on limite la pression lors de l'opération de mélangeage, par distillation de l'eau.

7. Procédé selon les revendications 1 à 5, caractérisé en ce qu'on effectue dans un réacteur clos le mélangeage du mélange contenant l'acide 3-sulfobenzoïque avec l'hydroxyde alcalin de manière à maintenir la pression résultante.

8. Procédé selon la revendication 7, caractérisé en ce qu'on utilise comme réacteur un tube mélangeur.

9. Procédé selon les revendications 1 à 8, caractérisé en ce qu'on effectue l'hydrolyse sous pression du mélange réactionnel alcalin sous la pression de vapeur d'eau particulière au système ou sous une pression partielle de vapeur d'eau d'environ 5 à 80 bars.

10. Procédé selon les revendications 1 à 9, caractérisé en ce qu'on acidifie jusqu'à un pH d'environ 0 à 4, à l'aide d'acides minéraux, le mélange obtenu après l'hydrolyse sous pression et on le dilue avec la quantité d'eau nécessaire pour la dissolution des sels minéraux.